# NEUE EUROPÄISCHE PATENTSCHRIFT

(11) **EP 1 933 726 B2**
(45) Veröffentlichungstag und Bekanntmachung des Hinweises auf die Entscheidung über den Einspruch: **20.11.2013**
(45) Hinweis auf die Patenterteilung: 25.08.2010
(21) Anmeldenummer: 06809496.0
(22) Anmeldetag: 04.10.2006
(51) Int. Cl.: A61B 17/16, A61B 17/28, A61B 17/32, E21B 17/20, F16C 1/04

(54) **FLEXIBLE HOHLWELLE**
FLEXIBLE HOLLOW SHAFT
ARBRE CREUX FLEXIBLE

(30) Priorität: 05.10.2005 CH 16052005
(43) Veröffentlichungstag der Anmeldung: 25.06.2008
(73) Patentinhaber: Da Rold, Orlando, 4500 Solothurn (CH)
(72) Erfinder: Da Rold, Orlando, 4500 Solothurn (CH)
(74) Vertreter: Fleck, Hermann-Josef
(86) Internationale Anmeldenummer: PCT/IB2006/053629
(87) Internationale Veröffentlichungsnummer: WO 2007/039875

(56) Entgegenhaltungen:
- EP-A1- 0 445 918
- EP-A1- 0 764 423
- EP-A1- 1 484 072
- US-A- 2 717 146
- US-A- 4 362 520
- US-A- 4 706 659
- US-A- 5 755 731

## Beschreibung

Die vorliegende Erfindung betrifft eine flexible Hohlwelle gemäss Oberbegriff des Patentanspruchs 1.

Eine flexible Hohlwelle dieser Art ist in der US 4,706,659 A gezeigt. Hierbei sind Einzelteile der Hohlwelle mit schwalbenschwanzförmigen Abschnitten mit Spiel ineinander gesetzt, um eine Biegung der Hohlwelle und ein erforderliches Drehmoment zu erreichen.

Hohlwellen als Antriebswellen können aus verschiedenen Gründen erwünscht sein. Zum einen bietet eine Hohlwelle im Vergleich zu einer Vollwelle bei gleichem Durchmesser annähernd gleiche Möglichkeiten für die Übertragung von Drehmomenten bei merklich weniger Gewicht. Ein anderer Grund liegt darin, dass man durch den Hohlkörper Material abtransportieren kann. Dies erst ermöglicht es, wirklich lange Löcher zu bohren. Das Bohrgut wird vom Werkzeug an der Spitze des Rohres z.B. mittels Spülwasser durch die Hohlwelle nach oben geschwemmt. Typische Anwendungen dieser Art sind denn auch die Tiefenbohrungen für Wasser oder Erdöl. Das Bohrgut wird durch das Zentrum des Rohres, an dessen Spitze sich das Bohrwerkzeug befindet, mit von oben zugebrachter Flüssigkeit, abgeführt.

Einen gänzlich anderen Einsatz finden Hohlwellen bei ganz andern Dimensionen in der arthroskopischen Operationstechnik. An einem Ende von zwei ineinander laufenden dünnwandigen Rohren werden chirurgische Schneidinstrumente angebracht, wie sie in WO 00/45713 oder in DE 44 22 426 vorgestellt werden. Solche Vorrichtungen werden in der Arthroskopie mit sehr gutem Erfolg eingesetzt indem sie je nach Anwendung und Einsatz mit verschiedenen Schneidköpfen, Schnittkanten und Öffnungen ausgestattet werden. Deren Beliebtheit in der Anwendung ist darauf zurück zu führen, dass mit minimalen operativen Eingriff und auf feine Weise, Korrekturen krankhafter oder unfallbedingter Veränderungen in Gelenken vorgenommen werden können, ohne grosse Verletzung von Gewebe und Muskeln zu verursachen. Ein typisches Instrument dieser Art ist in DE 44 22 426 vorgestellt.

Die Ausbildung von Schneidköpfen und Schneidgeometrien ist in vielen Schriften dargestellt und ausführlich beschrieben. Diesen chirurgischen Schneidinstrumenten liegt immer die Technik zugrunde, dass man das abgetragene Material durch das Rohr abführt.

Solange solche Rohre gerade sind, bietet dies sowohl für Bodenbohrungen als auch für die sehr viel feinere Anwendung in der Arthroskopie keine allzu grossen Probleme. Die Herstellung dünnwandiger Rohre im Bereich von wenigen Millimetern Durchmesser ist mit den heute bekannten Techniken und Materialien kein Problem. Nun hat sich aber der Wunsch manifestiert, nicht nur gerade sondern auch gebogene Instrumente zu haben mit denen "um die Ecke" gearbeitet werden kann. Ideen für die Art der Ausbildung solch gebogener Schneidinstrumente für die Chirurgie finden sich z.B. in EP 0 445 918. Für Erdbohrungen wurde bereits 1947 in US 2,515,365 eine Möglichkeit in dieser Richtung aufgezeigt. Der Nachteil der Konstruktion nach EP 0 445 918 ist darin zu suchen, dass das Instrument zwischen den Schlitzen, die eine flexible Biegung während der Drehung überhaupt zulassen, sehr viele "Brücken" aufweist, die bei jeder Umdrehung im Elastizitätsbereich gebogen und gestreckt werden. Bei den hohen Umdrehungszahlen von weit über 1'000 rpm/min für chirurgisch eingesetzte Hohlwellen sind die Biegewechselbelastungen auf die Brücken enorm. Bruch der Hohlwellen ist oft die Folge. Die nach diesem Prinzip gefertigten Vorrichtungen sind deshalb nicht zuverlässig. Der Bruch der oben beschriebenen "Brücken" wird genau dann eintreten, wenn das durch die flexible Hohlwelle getriebene Werkzeug mit grösserer Kraft bohren oder schneiden sollte.

Eine Möglichkeit diese Belastung der Brücken im Rahmen zu halten liegt natürlich darin, dass man den Winkel in dem die Biegung erfolgt möglichst klein hält. Dies hat zur Folge, dass der Radius der Kurve, die das mit einer solchen Hohlwelle ausgerüstete Instrument beschreiben kann, relativ gross sein muss. Das heisst anderseits, dass die Länge der Hohlwelle länger wird, je grösser der gewünschte Winkel sein muss den die Hohlwelle zwischen dem Antriebsrohr und dem Abtriebsrohr beschreiben soll. Enge Radien mit denen man also in Nischen rein kommt sind mit solchen Konstruktionen nicht erreichbar.

Dies hat der Anmelder von EP 0445 918 realisiert und er hat deshalb eine weitere Anmeldung EP 0 840 572 gemacht, die eine neue Art der Konstruktion zeigt. Er benützte für die Anwendung bei chirurgischen Schneidgeräten das mit Patent US 2,515,365 von Zublin seit 1947 bekannte Prinzip im Gebiet der Erdbohrungen. Es wird eine spiralförmig verlaufende schwalbenschwanzähnliche Anordnung der Schlitze gewählt. Probleme gibt es in der Praxis jedoch trotzdem. Zwar ist die Anzahl "Brücken", die der Biegewechselbelastung unterworfen werden deutlich reduziert, aber eben nicht eliminiert. Noch immer ist der realisierbare Mindestradius relativ gross. Er lässt für eine flexible Hohlwelle dieser Bauart in etwa ca. 30° Abwinklung zu, will man eine vernünftige Länge der Vorrichtung erhalten.

Ein anderes Problem stellt sich bei der Festigkeit solcher Vorrichtungen. Das Erfassen der Kräfte dieser komplexen Konstruktion ist sehr schwierig. Durch die schwalbenschwanzförmige, formschlüssige und spiralförmige Anordnung der Schlitze ist nicht klar, wo die hauptsächlich belasteten Stellen der Konstruktion zu suchen sind. Es ist demnach schwierig die Konstruktion an den richtigen Stellen den auftretenden Kräften entsprechend zu gestalten. Das resultiert in einer Unsicherheit für den Einsatz dieser flexiblen Hohlwelle. Es ist bekannt, dass die solchermassen ausgebildeten flexiblen Hohlwellen während des Einsatzes gebrochen sind.

Diese flexiblen Hohlwellen werden mit Drehzahlen von weit über 1'000 rpm betrieben. Sie laufen in umhüllenden Rohren. Das bedeutet für die festen "Brücken" weit über 1'000 Lastwechsel pro Minute. Ein Einsatz dauert schnell mal 10 bis 20 Minuten, so dass bis zu 50'000 Lastwechsel das Material ermüden. Metalle können solche Biegungen im elastischen Bereich nur in sehr beschränktem Masse mitmachen.

Die vorliegende Erfindung stellt sich nunmehr die Aufgabe eine flexible Hohlwelle zu bauen, mit der bei Radien von wenigen Zentimeter, Bogen von über 90° realisiert werden können, ohne dabei das Material einer wechselnden Biegebelastung auszusetzen. Diese Aufgabe muss auch erfüllt sein, wenn die Geräte mit Oszillationen von 1'000 bis 8'000 Richtungswechsel und Umdrehungszahlen für Vor- und Rücklauf von 1'000 bis 16'000 rpm Anwendung finden. Bisher bekannte Hohlwellenkonstruktionen bieten diese Möglichkeit nicht.

Diese Aufgabe löst die vorliegende flexible Welle mit den Merkmalen des Patentanspruches 1. Weitere erfindungsgemässe Merkmale gehen aus den abhängigen Ansprüchen hervor und deren Vorteile sind in der nachfolgenden Beschreibung erläutert.

In der Zeichnung zeigt:
- Fig 1: Flexible Hohlwelle mit einem Mittelrohrelement
- Fig 2: Mittelrohr Element
- Fig 3: Chirurgisches Schneidinstrument mit einer flexiblen Hohlwelle
- Fig 4: Anordnung der Schwalbenschwanz-Anschlüsse
- Fig 5: Flexible Hohlwelle mit mehreren Mittelrohrelementen
- Fig 6: Flexible Hohlwelle mit mehreren durch Stege verbundene Mittelrohrelemente.

Die Figuren stellen bevorzugte beispielhafte Ausführungsvorschläge dar, welche in der nachfolgenden Beschreibung erläutert werden.

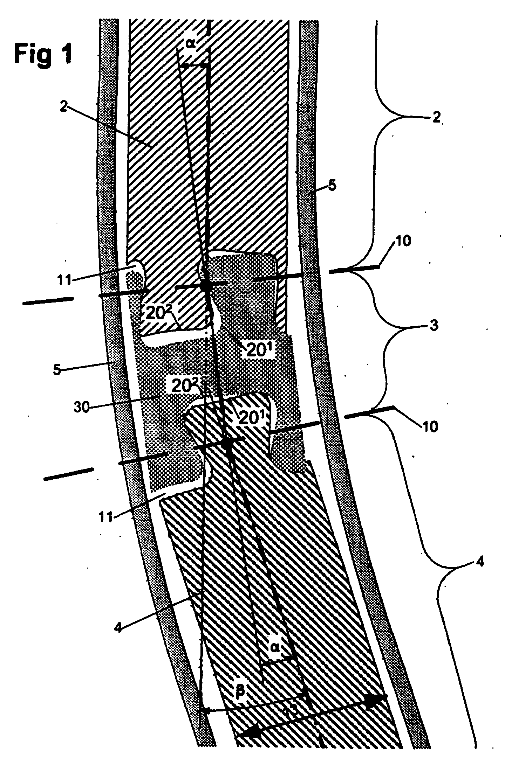

Fig 1 zeigt eine flexible Hohlwelle 1 mit einem Antriebsrohr 2, einem Mittelrohr 3 und einem Abtriebsrohr 4, sowie einem diese Elemente umhüllenden Führungsrohr 5. Diese Teile sind grundsätzlich ohne feste gegenseitige Verbindung, und sind als voneinander unabhängige und getrennte Teile ausgebildet.

Das Antriebsrohr 2 weist am einen Ende einen schwalbenschwanzförmigen Abschluss 20² auf. Am andern Ende befindet sich z.B. bei einem chirurgischen Schneidinstrument ein Anschlussteil an ein Antriebseinheit, die mittels Turbinenantrieb Drehzahlen von weit über 1'000 rpm erzeugt. Wird eine Hohlwelle der erfinderischen Art z.B. für Tiefenbohrungen eingesetzt, werden die Drehzahlen weit weniger hohe Umdrehungszahlen aufweisen.

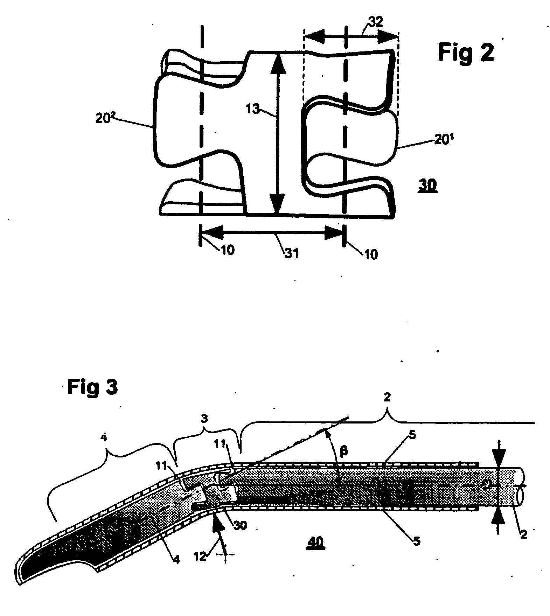

Das Abtriebsrohr 4 weist am einen Ende einen schwalbenschwanzförmigen Abschluss 20¹ auf. Als getriebener Teil der flexiblen Hohlwelle 1 ist an seinem andern Ende z.B. ein Werkzeug angebracht. Bei chirurgischen Schneidwerkzeugen sind das z.B. feine Messer wie in Fig 3 dargestellt. Werkzeuge für Tiefenbohrungen sind mit Bohrköpfen ausgerüstet, wie sie in dieser Branche üblich sind.

Versuche haben gezeigt, dass man das Mittelrohr 3 weglassen kann. Es stehen dann Antriebsrohr 2 und Abtriebsrohr 4 mit ihren gegenseitig formschlüssig passenden Abschlüssen 20² respektive 20¹ direkt im Eingriff und der erreichbare Winkel β entspricht dem erreichbaren Winkel α den man mit eine Trennung erreichen kann.

In der Regel ist jedoch ein Mittelrohr 3 aus mindestens einem Mittelrohrelement 30 zwischen Antriebsrohr 2 und Abtriebsrohr 4 eingefügt. Die Mittelrohrelemente 30 (Fig 2) weisen an einem Ende einen schwalbenschwanzförmigen Abschluss 20¹ und am andern Ende einen schwalbenschwanzförmigen Abschluss 20² auf. Jeder schwalbenschwanzförmige Abschluss 20¹ passt in seiner Form auf einen schwalbenschwanzförmigen Abschluss 20², wobei eine grob formschlüssige Verbindung mit Spiel 11 entsteht, über die ein Drehmoment übertragen werden kann.

Die schwalbenschwanzförmigen Abschlüsse 20² und 20¹ formieren sich um eine theoretische Trennstelle 10. Die praktisch vorhandenen Trennlinien winden sich schwalbenschwanzförmig dem Umfang der Rohre entlang und um diese theoretische Trennstelle 10. Zwischen den praktischen Trennstellen, den schwalbenschwanzförmigen Abschlüssen 20² und 20¹ wird je nach den gegebenen Bedürfnissen ein Spiel 11 von 0.01 und 0.5 mm vorgesehen. Dies ermöglicht, dass die Längsachsen der einzelnen Elemente, Antriebsrohr 2, Mittelrohr 3 und Abtriebsrohr 4 in einem Winkel α zueinander stehen können. Wenn sich nun die Hohlwelle dreht, "wandert" das Spiel 11 am Umfang der schwalbenschwanzförmigen Abschlüsse 20² und 20¹ entlang und lässt es auf diese Art zu, dass die Achsen im Winkel α zueinander drehen, während die Kraftübertragung zwischen den Elementen gewährleistet ist. Nicht eine Elastizität oder gar Verformung des Metalles, sondern das in der Trennung der schwalbenschwanzförmigen Abschlüsse 20² und 20¹ der theoretische Trennstelle 10 dem Umfang entlang "wandernde" Spiel 11 ermöglicht die resultierende "flexible Biegung" der Hohlwelle 1 über das Mittelrohr 3 und gewährleistet die erwünschte Kraftübertragung.

Damit die einzelnen Elemente geführt sind, werden alle Elemente der flexiblen Hohlwelle 1 durch das Führungsrohr 5 gehalten, gelagert und geführt. Die ganze aus flexibler Hohlwelle 1 und Führungsrohr 5 bestehende Einheit 40 (Fig 3) ist um einen festen Winkel β gekrümmt. Dieser Winkel β den das Gerät nach dessen Fertigstellung beschreibt, ist fest und nicht flexibel. Die im Führungsrohr 5 geführte flexible Hohlwelle 1 ermöglicht aber die Übertragung eines Drehmomentes "um die Kurve". Fig 3 zeigt die Anwendung an einem chirurgischen Schneidinstrument 40. Die flexible Hohlwelle 1 der vorgestellten Art kann Winkel bis über 90° bei kleinen Radien 12 von z.B. 5 - 10 cm und Drehzahlen von weit über 1'000 rpm überbrücken.

Eine Leistung, die nur durch die vorgestellte Konstruktion einer flexiblen Hohlwelle erreicht werden kann. Man erreicht dies, indem die Länge 31 (Fig 2) der Mittelrohrelemente 30 und die Höhe 32 der schwalbenschwanzförmigen Abschlüsse 20 angepasst werden. Mit einer Vielzahl von kurzen Mittelrohrelementen 30 die kleine Höhen 32 der schwalbenschwanzförmigen Abschlüsse 20 aufweisen, können kleine Radien 12 erreicht werden. Aus mechanischen Gründen ist allerdings die minimale Grösse eines Mittelrohrelementes 30 je nach Durchmesser 13 der flexiblen Hohlwelle 1 (Fig 2) begrenzt. Zu beachten ist auch, dass die Grössenordnung des zu überbrückenden Winkels β von der Anzahl eingesetzter Mittelrohrelementen abhängt. Je mehr Mittelrohrelemente 30 eingesetzt werden, desto grösser wird der Winkel β, da er sich aus den einzelnen Winkeln α zwischen den einzelnen Elementen zusammensetzt.

Die Anordnung der Schwalbenschwanzformen über den Umfang der Hohlwelle ist möglichst zufällig. Es besteht jedoch die Bedingung, dass immer ein Abschluss 20¹ auf einen Abschluss 20² passen muss, wobei die Abschlüsse 20¹ von Abtriebsrohr 4 und 20² von Antriebsrohr 2 jeweils als 20¹ resp. 20² ausgebildet sind und auf die Abschlüsse 20² resp. 20¹ der anschliessenden Mittelrohrelemente 30 passen müssen.

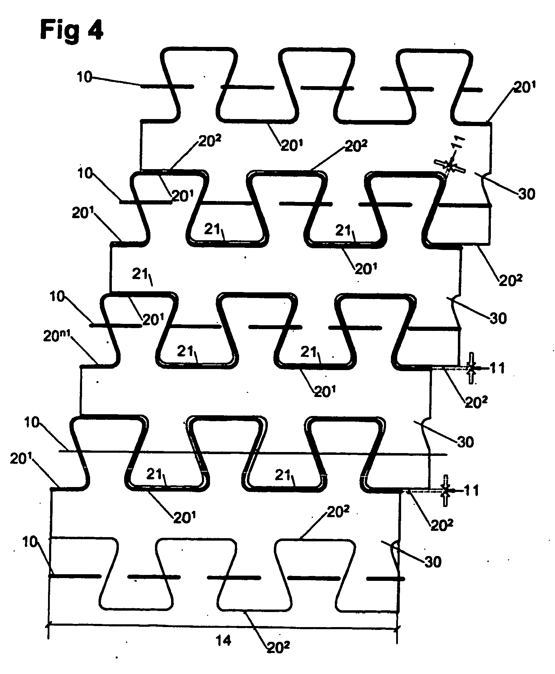

Eine Möglichkeit der Anordnung der schwalbenschwanzförmigen Abschlüsse 20¹ und 20² zeigt Fig 4. Die Darstellung zeigt eine Abwicklung der einzelnen Mittelrohrelementen 30 über den Umfang 14, der 3.14 mal dem Durchmesser 13 der Rohre entspricht: Die Anordnungen können beliebig sein. Eine Notwendigkeit besteht darin, dass die Abschlüsse 20¹ und 20² jeweils paarweise aufeinander passen müssen. In eben dieser Darstellung wird auch dargestellt, wie das Spiel 11 zwischen den einzelnen Mittelrohrelementen 30 in den paarweisen Abschlüssen 20¹ und 20² gebildet wird. Die Abschlüsse 20¹ resp. 20² von Abtriebsrohr 4 resp. Antriebsrohr 2 bilden sinngemäss ein Paar mit den Abschlüssen 20¹ resp. 20² der anschliessenden Mittelrohrelementen 30.

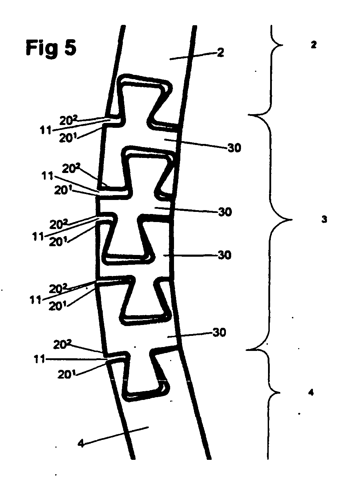

In Fig 5 wird die flexible Hohlwelle 1 mit dem aus mehreren Mittelrohrelementen 30 bestehenden Mittelrohr 3, dem Antriebsrohr 2 und dem Abtriebsrohr 4 in der Zusammenstellung gezeigt. Um einen ruhigeren Lauf während des Einsatzes der flexiblen Hohlwelle 1 zu erreichen, werden die schwalbenschwanzförmigen Trennlinien 20 der Mittelrohrelemente 30 nicht - wie in Fig 4 dargestellt - regelmässig am Umfang verteilt, sondern in zufälliger Weise angeordnet, wie in Fig 5 dargestellt.

Ein optimales Spiel 11 zwischen Antriebsrohr 2, Abtriebsrohr 4 und den Mittelrohrelementen 30 wird dadurch erreicht, dass man die Hohlwelle in einem dafür speziell vorgesehenen Führungsrohr 5 einlaufen lässt. Tests haben gezeigt, dass durch diese Art des Einlaufens das Geräusch das die flexible Hohlwelle 1 während des Betriebs erzeugt erheblich vermindert wird. Gleichzeitig wird sich auch das Spiel 11 zwischen allen paarweise im Eingriff stehenden Abschlüsse 20¹ und 20² der Lage angepasst einstellen.

Die bisher vorgestellte Hohlwelle 1 weist zwischen Mittelrohrelementen 30, resp. zwischen Mittelrohrelementen 30, Antriebsrohr 2 und Abtriebsrohr 4 ein Spiel 11 von 0.01 bis 05mm auf. Dieses Spiel 11 ermöglicht erst die Drehung der Hohlwelle 1 im Führungsrohr 5. Allerdings ergibt sich dadurch der Nachteil, dass die durch Absaugung erreichte Rückspülung durch die Hohlwelle 1 mit den durch das Spiel 11 am Aussendurchmesser des Bogens in gekrümmter Lage Öffnungen entstehen, welche die Absaugung mindestens behindern, wenn nicht gar verunmöglichen. Um dies zu eliminieren gibt es verschiedene Methoden, die im Versuch technisch erprobt wurden und ausgeführt werden.

Man kann die flexible Hohlwelle 1 vor dem Einführen in das Führungsrohr 5 in eine Kunststoffschmelze tauchen, so dass die Teile der Hohlwelle 1 mit einem flexiblen Kunststoffbelag überzogen werden. Da es sich beim Spiel 11 um Öffnungen im bereich von Zehntels Millimeter handelt, kann für diesen Zweck ein durchschnittlich elastisches Material eingesetzt werden, das an dem Material der Hohlwelle haftet. Eine weitere Möglichkeit liegt darin, dass man in die Hohlwelle 1 einen aus elastischem Material bestehenden Schlauch einführt, und durch ein Dehnwerkzeug presst, so dass das Material des Schlauches am Material der Hohlwelle haftet. Das Dehnwerkzeug kann eine aufblasbare ballonartige Vorrichtung sein, ähnlich solcher die für die Dehnung verengter Herzkranzgefässe Verwendung finden.

Die wohl einfachste Methode besteht darin, dass man einen Schlauch um die Hohlwelle anbringt und dann mittels Schrumpfen um den Aussendurchmesser der Hohlwelle hüllt. Diese Methode hat den Vorteil, dass unabhängig der Materialwahl von Schlauch und Hohlwelle das in der Art aufgeschrumpfte Material durch die Eigenelastizität auf der Hohlwelle haftet. Die Hohlwelle 1 kann durch jede dieser oben beschriebenen Massnahmen wirkungsvoll abgedichtet werden.

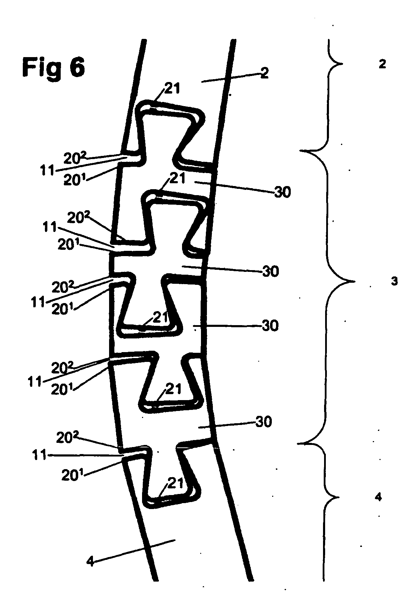

Für die Herstellung der Hohlwelle 1 kann eine spezielle Methode verwendet werden die den Herstellungsprozess und vor allem die Handhabung der Hohlwelle während des Herstellungsprozesses vereinfacht: Wie in Fig 4 dargestellt, werden Stege 21 zwischen den einzelnen Teilen der Hohlwelle 1 eingebaut. Diese werden so ausgelegt, dass sie bei der ersten Biegung der Hohlwelle 1 die im Führungsrohr 5 erfolgen soll, brechen ohne an den Teilen der Hohlwelle 1 Spuren zu hinterlassen. In Fig 6 ist gezeigt, wie die Reste der gebrochenen Stege 21 an den Teilen der Hohlwelle verbleiben. Die Anwendung dieser Methode bietet den Vorteil, dass im Produktionsprozess durchwegs mit geraden, zylindrischen Hohlwellen 1 gearbeitet werden kann. So können z.B. Oberflächenbehandlungen der bereits getrennten Teile dadurch einfacher erfolgen, dass die Hohlwelle 1 eine gerade und stabile Form hat. Wären die Teile 2,30 und 4 bereits in dieser Phase voneinander unabhängig, ist die Bearbeitung dieser lose ineinander gehaltenen Teile 2,30 und 4 schwierig. Speziell auch für das Einbringen oder Umhüllen der Hohlwelle 1 mit einem flexiblen und elastischen Schlauch würde sehr viel schwieriger.

Das Einbringen der Hohlwelle 1 in das Führungsrohr 5 erfolgt in diesem Fall in der geraden durch die Stege 21 gehaltenen Form der Hohlwelle 1. Sobald nun eine Krümmung der Hohlwelle 1 erfolgt, werden die Sollbruchstellen brechen. Spätestens brechen sie aber, wenn die als Antriebswelle eingesetzte Hohlwelle 1 sich mit hoher Drehzahl im gekrümmten Führungsrohr 5 zu drehen beginnt.

Die Herstellung solcher Stege 21 als Sollbruchstellen stellt für die heute einsetzbaren Herstelltechniken kein Problem dar. Alle Herstellungsmethoden wie Laserschneiden, Erodieren oder Ätzen lassen es zu, dass man Stege 21 nicht nur stehen lässt, sondern diese auch auf die für den Einsatz als Sollbruchstellen notwendige Dimensionierung bringt.

## Patentansprüche

1. Flexible Hohlwelle, die sich aus einem Antriebsrohr (2), einem Mittelrohr (3) aus mindestens einem Mittelrohrelement (30), einem Abtriebsrohr (4) und einem Führungsrohr (5) zusammensetzt, wobei das Antriebsrohr (2), das Mittelrohr (3) und das Abtriebsrohr (4) voneinander getrennte unabhängige Einzelteile sind, wobei das Antriebsrohr (2) an einem Ende einen zweiten schwalbenschwanzförmigen Abschluss (20²) aufweist, das Abtriebsrohr (4) an einem Ende einen ersten schwalbenschwanzförmigen Abschluas (20¹) aufweist und das mindestens eine Mittelrohrelement (30) an einem Ende einen ersten schwalbenschwanzförmigen Abschluss (20¹) und am anderen Ende einen zweiten schwalbenschwanzförmigen Abschluss (20²) aufweist, wobei jeder erste schwalben-schwanzförmige Abschluss (20¹) in seiner Form auf einen zweiten schwalbenschwanzförmigen Abschluss (20²) passt und wobei eine formschlüssige Verbindung mit Spiel (11) entsteht, über die ein Drehmoment übertragbar ist,
**dadurch gekennzeichnet,**
**dass** die einzelnen Teile Antriebsrohr (2), alle Mittelrohrelemente (30) und das Abtriebsrohr (4) im Produktionsprozess zu einer geraden zylindrischen Hohlwelle mit Stegen (21) verbunden sind wobei diese Stege (21) so ausgelegt sind, dass sie bei kleiner Krafteinwirkung bei der ersten Biegung der Hohlwelle (1) im Führungsrohr (5) brechen, ohne an den Einzelteilen Spuren zu hinterlassen, so dass das Antriebsrohr (2), die Mittelrohrelemente (30) und das Abtriebsrohr (4) anlässlich der ersten Drehung der Hohlwelle (1) in dem gebogenen Führungsrohr (5) unabhängige, getrennte Teile ohne feste gegenseitige Verbindung sind.

2. Flexible Hohlwelle nach Anspruch 1,
**dadurch gekennzeichnet,**
**dass** das Antriebsrohr (2), das Mittelrohr (3) und das Abtriebsrohr (4) in dem Führungsrohr (5) gehalten und geführt sind.

3. Flexible Hohlwelle nach Anspruch 1,
**dadurch gekennzeichnet,**
**dass** die Mittelrohrelemente (30) am einen Ende auf einer senkrecht zur Achse am Umfang verlaufenden theoretischen Trennlinie (10) einen umgreifenden ersten schwalbenschwanzförmigen Abschluss (20ⁿ¹) aufweisen und am andem Ende einen auf den ersten schwalbenschwanzförmigen Abschluss (20ⁿ¹) eines nächsten Mittelrohrelemente (30) passenden zweiten schwalbenschwanzförmigen Abschluss (20ⁿ²) aufweisen.

4. Flexible Hohlwelle nach Anspruch 1,
**dadurch gekennzeichnet,**
**dass** das Antriebsrohr (2) auf der dem Mittelrohr (3) zugewandten Seite auf einer senkrecht zur Achse am Umfang verlaufenden theoretischen Trennlinie (10) einen diese theoretische Trennlinie (10) umgreifenden zweiten schwalbenschwanzförmigen Abschluss (20²) bildet.

5. Flexible Hohlwelle nach Anspruch 1,
**dadurch gekennzeichnet,**
**dass** das Abtriebsrohr (4) auf der dem Mittelrohr (3) zugewandten Seite auf einer senkrecht zur Achse am Umfang verlaufenden theoretischen Trennlinie (10) einen diese theoretische Trennlinie (10) umgreifenden ersten schwalbenschwanzförmigen Abschluss (20¹) bildet.

6. Flexible Hohlwelle nach Anspruch 4,
**dadurch gekennzeichnet,**
**dass** der schwalbenschwanzförmige Abschluss (20²) des Antriebsrohres (2) formgleich passend ist mit dem ersten schwalbenschwanzförmigen Abschluss (20ⁿ¹) eines ersten Mittelrohrelementes (30).

7. Flexible Hohlwelle nach Anspruch 5,
**dadurch gekennzeichnet,**
**dass** der schwalbenschwanzförmige Abschluss (20¹) des Abtriebsrohres (4) formgleich passend ist mit dem zweiten schwalbenschwanzförmigen Abschluss (20ⁿ²) eines letzten Mittelrohrelementes (30).

8. Flexible Hohlwelle nach Anspruch 1,
**dadurch gekennzeichnet,**
**dass** der zweite schwalbenschwanzförmige Abschluss (20ⁿ²) eines Mittelrohr-elements (30) formgleich passend ist mit dem ersten schwalbenschwanzför migen Abschluss (20ⁿ¹) eines nächsten Mittelrohrelements (30).

9. Flexible Hohlwelle nach einem der Ansprüche 3 bis 8,
**dadurch gekennzeichnet,**
**dass** zwischen allen ineinander greifenden schwalbenschwanzförmigen Abschlüssen (20²) des Antriebsrohres (2), den Abschlüssen (20ⁿ¹, 20ⁿ²) der Mittelrohrelemente (30) und dem Abschluss (20¹) des Abtriebsrohres (4), jeweils ein Spiel (11) von 0,01 bis 0,5 mm vorhanden ist.

10. Flexible Hohlwelle nach Anspruch 1,
**dadurch gekennzeichnet,**
**dass** die flexible Hohlwelle (1) einen das Antriebsrohr (2), die Mittelrohrelemente (30) und das Abtriebsrohr (4) erfassenden, und diese Teile verbindenden Kunststoffbelag aufweist.

11. Flexible Hohlwelle nach Anspruch 1,
**dadurch gekennzeichnet,**
**dass** in der flexiblen Hohlwelle (1), also im Antriebsrohr (2), im Mittelrohr (3) und im Abtriebsrohr (4) ein alle diese Teile im Innendurchmesser durchlaufender dünner Kunststoffschlauch formschlüssig eingepasst ist.

12. Flexible Hohlwelle nach Anspruch 9,
**dadurch gekennzeichnet,**
**dass** über die flexible Hohlwelle (1), also über dem Antriebsrohr (2), über dem Mittelrohr (3) und über dem Abtriebsrohr (4) ein alle diese Teile umhüllender Kunststoffschlauch übergestülpt ist, der sich zwischen den Teilen der Hohlwelle (1) und dem Führungsrohr (5) befindet und mit den Teilen der Hohlwelle (1) fest verbunden ist.

## Claims

1. Flexible hollow shaft which is composed of a drive tube (2), a centre tube (3) consisting of at least one middle tube element (30), an output tube (4) and a guide tube (5), the drive tube (2), the centre tube (3) and the output tube (4) being independent individual parts which are separate from one another, the drive tube (2) having at one end a second dovetail-shaped termination (202), the output tube (4) having at one end a first dovetail-shaped termination (201) and the at least one middle tube element (30) having at one end a first dovetail-shaped termination (201) and having at the other end a second dovetail-shaped termination (202), the form of each first dovetail-shaped termination (201) matching a second dovetail-shaped termination (202) and creating a form fit connection with play (11) for transmitting torque,
**characterized in that**,
the individual parts of the drive tube (2), all centre-tube elements (30) and the output tube (4) are connected in the production process to a straight cylindrical hollow shaft by webs (21), the said webs (21) designed to break if a small force is applied when the hollow shaft (1) is bent the first time in the guide tube (5) without leaving any residue on the individual parts, with the result that the drive tube (2), the centre-tube elements (30) and the output tube (4) are separated independent parts without any fixed connection to each other on the occasion of the first rotation of the hollow shaft (1) in the bent guide tube (5).

2. The flexible hollow shaft as claimed in claim 1, **characterized in that**, the drive tube (2), the middle tube (3) and the driven tube (4) are held and guided in the guide tube (5).

3. The flexible hollow shaft as claimed in claim 1, **characterized in that**, the middle tube elements (30) have at one end, on the theoretical separating line (10) running perpendicularly with respect to the axis on the circumference, a first surrounding dovetail-shaped termination (20n1) and have at the other end a second dovetail-shaped termination (20n2) fitting on to the first dovetail-shaped termination (20n1) of a next middle tube element (30).

4. The flexible hollow shaft as claimed in claim 1, **characterized in that**, the drive tube (2), on the side facing the middle tube (3), forms, on a theoretical separating line (10) running perpendicularly with respect to the axis on the circumference, a second dovetail-shaped termination (202) surrounding this theoretical separating line (10).

5. The flexible hollow shaft as claimed in claim 1, **characterized in that**, the drive tube (4), on the side facing the middle tube (3), forms, on a theoretical separating line (10) running perpendicularly with respect to the axis on the circumference, a first dovetail-shaped termination (201) surrounding this theoretical separating line (10).

6. The flexible hollow shaft as claimed in claim 4, **characterized in that**, the dovetail-shaped termination (202) of the drive tube (2) fits identically in form with the first dovetail-shaped termination (20n1) of a first middle tube element (30).

7. The flexible hollow shaft as claimed in claim 5, **characterized in that**, the dovetail-shaped termination (201) of the driven tube (4) fits in identical form with the second dovetail-shaped termination (20n2) of a last middle tube element (30).

8. The flexible hollow shaft as claimed in claim 1, **characterized in that**, the second dovetail-shaped termination (20n2) of one middle tube (30) fits in identical form with the first dovetail-shaped termination (20n1) of a next middle tube element (30).

9. The flexible hollow shaft as claimed in claims 3 to 8, **characterized in that**, a play (11) of 0.01 to 0.5 mm is present in each case between all the dovetail-shaped terminations (202) of the drive tube (2) which engage one in the other, the terminations (20n1, 20n2) of the middle tube elements (30) and the termination (201) of the drive tube (4).

10. The flexible hollow shaft as claimed in claim 1, **characterized in that**, the flexible hollow shaft (1) has a plastic coating covering the drive tube (2), the middle tube elements (30) and the driven tube (4) and connecting these parts.

11. The flexible hollow shaft as claimed in claim 1, **characterized in that**, in the flexible hollow shaft (1), that is to say the drive tube (2), in the middle tube (3) and in the driven tube (4), is fitted with a form fit a thin plastic hose running through all these parts in the inside diameter.

12. The flexible hollow shaft as claimed in claim 9, **characterized in that**, over the flexible hollow shaft (1), that is to say over the drive tube (2), over the middle tube (3) and over the driven tube (4), is slipped a plastic hose which encases all these parts and which is located between the parts of the hollow shaft (1) and the drive tube (5) and is fixedly connected to the parts of the hollow shaft (1).

## Revendications

1. Arbre creux flexible, qui se compose d'un tube d'entraînement (2), d'un tube central (3) qui se compose d'au moins un élément de tube central (30), d'un tube de sortie (4) et d'un tube de guidage (5), le tube d'entraînement (2), le tube central (3) et le tube de sortie (4) étant des pièces détachées indépendantes séparées les unes des autres, le tube d'entraînement (2) comprenant, à une extrémité, une deuxième terminaison (20²) en forme de queue d'aronde, le tube de sortie (4) comprenant, à une extrémité, une première terminaison (20¹) en forme de queue d'aronde et l'au moins un élément de tube central (30) comprenant, à une extrémité, une première terminaison (20¹) en forme de queue d'aronde et, à l'autre extrémité, une deuxième terminaison (20²) en forme de queue d'aronde, chaque première terminaison (20¹) en forme de queue d'aronde étant adaptée par sa forme identique à une deuxième terminaison (20²) en forme de queue d'aronde et une connexion par engagement par correspondance géométrique avec jeu (11) étant produite, par le biais de laquelle un couple peut être transmis,
**caractérisé en ce que**
les pièces séparées du tube d'entraînement (2), de tous les eléments de tube central (30) et du tube de sortie (4) sont connectées par des nervures (21) lors du processus de production pour former un arbre creux rectiligne cylindrique, ces nervures (21) étant conçues pour se casser sous l'application d'une faible force pendant le premier cintrage de l'arbre creux (1) dans le tube de guidage (5), sans laisser de traces au niveau des pièces individuelles, de sorte que le tube d'entraînement (2), les éléments de tube central (30) et le tube de sortie (4) soient des pièces indépendantes séparées sans connexion mutuelle fixe dès la première rotation de l'arbre creux (1) dans le tube de guidage cintré (5).

2. Arbre creux flexible selon la revendication 1,
**caractérisé en ce que**
le tube d'entraînement (2), le tube central (3) et le tube de sortie (4) sont maintenus et guidés dans le tube de guidage (5).

3. Arbre creux flexible selon la revendication 1,
**caractérisé en ce que**
les éléments de tube central (30) présentent, à une extrémité, sur une ligne de séparation théorique (10) s'étendant sur la périphérie perpendiculairement à l'axe, une première terminaison d'engagement périphérique (20ⁿ¹) en forme de queue d'aronde, et présentent, à l'autre extrémité, une deuxième terminaison (20ⁿ²) s'adaptant à la première terminaison (20ⁿ¹) en forme de queue d'aronde d'un élément de tube central suivant (30).

4. Arbre creux flexible selon la revendication 1,
**caractérisé en ce que**
le tube d'entraînement (2) forme, sur le côté tourné vers le tube central (3), sur une ligne de séparation théorique (10) s'étendant sur la périphérie perpendiculairement à l'axe, une deuxième terminaison (20²) en forme de queue d'aronde pour l'engagement périphérique de cette ligne de séparation théorique (10).

5. Arbre creux flexible selon la revendication 1,
**caractérisé en ce que**
le tube de sortie (4) forme sur le côté tourné vers le tube central (3), sur une ligne de séparation théorique (10) s'étendant sur la périphérie perpendiculairement à l'axe, une première terminaison (20¹) en forme de queue d'aronde pour l'engagement périphérique de cette ligne de séparation théorique (10).

6. Arbre creux flexible selon la revendication 4,
**caractérisé en ce que**
la terminaison (20²) en forme de queue d'aronde du tube d'entraînement (2) est adaptée par sa forme identique à la première terminaison (20ⁿ¹) en forme de queue d'aronde d'un premier élément de tube central (30).

7. Arbre creux flexible selon la revendication 5,
**caractérisé en ce que**
la terminaison (20¹) en forme de queue d'aronde du tube de sortie (4) est adaptée par sa forme identique à la deuxième terminaison (20ⁿ²) en forme de queue d'aronde d'un dernier élément de tube central (30).

8. Arbre creux flexible selon la revendication 1, **caractérisé en ce que** la deuxième terminaison (20ⁿ²) en forme de queue d'aronde d'un élément de tube central (30) est adaptée par sa forme identique à la première terminaison (20ⁿ¹) en forme de queue d'aronde d'un élément de tube central (30) suivant.

9. Arbre creux flexible selon l'une quelconque des revendications 3 à 8, **caractérisé en ce que**
l'on prévoit entre toutes les terminaisons (20²) en forme de queue d'aronde, venant en prise les unes dans les autres, du tube d'entraînement (2), les terminaisons (20ⁿ¹, 20ⁿ²) des éléments de tube central (30) et la terminaison (20¹) du tube de sortie (4), à chaque fois un jeu (11) de 0,01 à 0,5 mm.

10. Arbre creux flexible selon la revendication 1,
**caractérisé en ce que**
l'arbre creux flexible (1) présente une garniture en plastique saisissant le tube d'entraînement (2), les éléments de tube central (30) et le tube de sortie (4) et reliant entre elles ces pièces.

11. Arbre creux flexible selon la revendication 1,
**caractérisé en ce que**
dans l'arbre creux flexible (1), c'est-à-dire dans le tube d'entraînement (2), dans le tube central (3) et dans le tube de sortie (4) est adapté, par engagement par correspondance géométrique, un tuyau en plastique mince traversant toutes ces pièces dans leur diamètre intérieur.

12. Arbre creux flexible selon la revendication 9,
**caractérisé en ce que**
par-dessus l'arbre creux flexible (1), c'est-à-dire par-dessus le tube d'entraînement (2), le tube central (3) et le tube de sortie (4), est enfoncé un tuyau en plastique enveloppant toutes ces pièces, lequel se trouve entre les pièces de l'arbre creux (1) et du tube de guidage (5), et est connecté fixement aux pièces de l'arbre creux (1).
